(19) Europäisches Patentamt
European Patent Office
Office européen des brevets

(11) Veröffentlichungsnummer: **0 275 520 B1**

# (12) EUROPÄISCHE PATENTSCHRIFT

(45) Veröffentlichungstag der Patentschrift: **11.12.91**

(51) Int. Cl.[5]: **C07D 471/04**, A01N 43/90, //(C07D471/04,221:00,221:00)

(21) Anmeldenummer: **87118899.1**

(22) Anmeldetag: **19.12.87**

Die Akte enthält technische Angaben, die nach dem Eingang der Anmeldung eingereicht wurden und die nicht in dieser Patentschrift enthalten sind.

## (54) Substituierte 1,8-Naphthyridin-Derivate und diese enthaltende Fungizide.

(30) Priorität: **31.12.86 DE 3644825**

(43) Veröffentlichungstag der Anmeldung: **27.07.88 Patentblatt 88/30**

(45) Bekanntmachung des Hinweises auf die Patenterteilung: **11.12.91 Patentblatt 91/50**

(84) Benannte Vertragsstaaten: **AT BE CH DE ES FR GB GR IT LI NL SE**

(56) Entgegenhaltungen:
**DE-A- 2 918 591**
**GB-A- 1 432 967**
**GB-A- 1 496 371**

**JOURNAL OF THE AMERICAN CHEMICAL SOCIETY, PERKIN TRANSACTIONS I, 1977, Seiten 789-795, London, GB; I. HERMECZ et al.: "Nitrogen Bridgehead Compounds. Part 4. 1-3N-C-Acyl Migration. Part 2"**

(73) Patentinhaber: **BASF Aktiengesellschaft**
**Carl-Bosch-Strasse 38**
**W-6700 Ludwigshafen(DE)**

(72) Erfinder: **Graf, Hermann, Dr.**
**Ginsterstrasse 15**
**W-6704 Mutterstadt(DE)**
Erfinder: **Franz, Lothar, Dr.**
**Sternstrasse 197**
**W-6700 Ludwigshafen(DE)**
Erfinder: **Sauter, Hubert, Dr.**
**Neckarpromenade 20**
**W-6800 Mannheim 1(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 275 520 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

CHEMICAL ABSTRACTS, Band 77, Nr. 21, 20. November 1972, Seite 446, Abstract Nr. 140016z, Columbus, Ohio, US; R. DOMORI et al.: "Aminonaphthyridines"; & JP - A - 72 29519

CHEMICAL ABSTRACTS, Band 86, Nr. 13, 28. März 1977, Seite 534, Abstract Nr. 89704d, Columbus, Ohio, US; O. LIVI et al.: "Synthesis and biological activity of 1,2,3-triazolo-1,8-naphthyridine derivatives"

CHEMICAL ABSTRACTS, Band 87, Nr. 19, 7. November 1977, Seite 593, Abstract Nr. 152164r, Columbus, Ohio, US; M. WOZNIAK et al.: "2-Hydroxy-4-hydrazino-1,8-naphthyridine"

CHEMICAL ABSTRACTS, Band 85, Nr. 3, 19. Juli 1976, Seite 673, Abstract Nr. 21166d, Columbus, Ohio, US; W. CZUBA et al.: "Differences in the reactivity from nucleophilic substitution of halogens in position 2 and 4 of 1,8-naphthyridine"

CHEMICAL ABSTRACTS, Band 101, Nr. 15, 8. Oktober 1984, Seite 702, Abstract Nr. 130611q, Columbus, Ohio, US; G.B. BARLIN et al.: "Potential antimalarials. I. 1,8-Naphthyridines"

## Beschreibung

Die vorliegende Erfindung betrifft neue 1,8-Naphthyridin-Derivate mit fungizider Wirkung, diese enthaltende Fungizide und Verfahren zu ihrer Herstellung.

Es ist bekannt, N-Trichlormethylthio-tetrahydro-phthalimid (Chemical Week, June 21, 1972, Seitz 46) als Fungizid zu verwenden. Außerdem sind die Verbindungen 2,7-Dimethyl-4-hydroxy-1,8-naphthyridin (Hermecz et al, J.Chem. Soc.Perkin Trans. I, 1977, Seite 789) und 2,7-Dimethyl-4-chlor-1, 8-naphthyridin (J.Het.Chem. 20, 1053-1056 (1983)) bekannt. Es sind ferner 1,4-Dihydro-1,8-naphthyridin-4-one (GB-A-1 432 967), 3-Aminonaphthyridine (C.A. 77 (1972), 1 400 163), Triazolyl-4-aminonaphthyridine mit fungizider Wirkung (C.A. 86 (1977), 89 704 d), substituierte 4-Amino-tetrahydrochinolinemit fungizider Wirkung (DE-A-2 918 591), substituierte 4-Aminochinoline mit fungizider Wirkung (GB-A-1 496 371), 2-Hydroxy-4-hydrazino-1,8-naphthyridine (C.A. 87 (1977), 152 146 r), 4-Hydrozin-1,8-naphthyridine (C.A. 85 (1976), 21 166 d) und 4-Amino-1,8-naphthyridine (C.A. 101 (1984), 1 306 119) bekannt.

Es wurde nun gefunden, daß 1 ,8-Naphthyridin-Derivate der Formel I

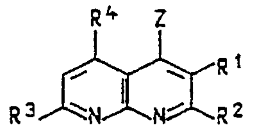

I,

worin

- $R^1$ einen $C_4$- bis $C_{12}$-Alkyl- oder $C_4$- bis $C_{12}$-Alkoxy-$C_1$-$C_4$-alkyl-rest bedeutet,
- $R^2$ und $R^3$ unabhängig voneinander $C_1$- bis $C_3$-Alkyl,
- $R^4$ Wasserstoff oder $C_1$-$C_4$-Alkyl,
- Z $NH_2$ oder $NH$-$NR^6R^5$,
- $R^5$ und $R^6$ unabhängig voneinander Wasserstoff, $C_1$-$C_4$-Alkyl, Formyl oder $C_1$-$C_4$-Alkyl-CO-

bedeuten, bekannte Verbindungen in ihrer fungiziden Wirkung gegen pflanzenpathogene Pilze übertreffen.

$C_4$-$C_{12}$-Alkyl ist beispielsweise gerades oder verzweigtes Butyl, Pentyl, Hexyl, Heptyl, Octyl, Nonyl, Decyl, Undecyl, Dodecyl.

$C_4$-$C_{12}$-Alkoxy ist beispielsweise gerades oder verzweigtes Butoxy, Pentoxy, Hexoxy, Heptoxy, Octyloxy, Nonyloxy, Decyloxy, Dodecyloxy. $C_1$-$C_4$-Alkyl ist beispielsweise gerades oder verzweigtes Methyl, Ethyl, Propyl, Butyl.

$C_1$-$C_4$-Alkyl-CO ist beispielsweise Acetyl, Propionyl, Butyryl.

Die Herstellung der neuen Verbindungen kann beispielsweise wie folgt vorgenommen werden:
4-Hydroxy-1,8-Naphthyridine der Formel Va, b,

Va

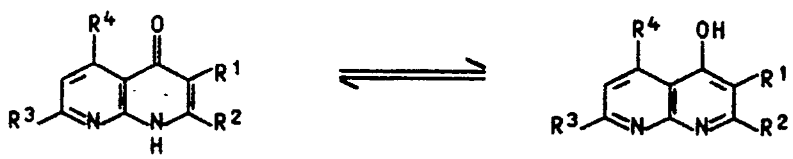

Vb

wobei diese Verbindungen überwiegend in der Form Va vorliegen, erhält man, indem man $\beta$-Ketoester der Formel II

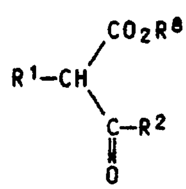

II,

mit einem entsprechenden 2-Amino-pyridin der Formel III

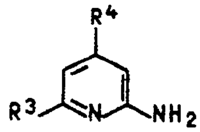

III,

wobei $R^8$ für einen $C_1$-$C_4$-Alkylrest steht und $R^1$ bis $R^4$ die oben genannte Bedeutung besitzen, zu 4H-Pyrido[1,2-a]pyrimidin-4-onen der Formel IV

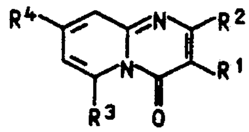

IV,

umsetzt und diese zu 4-Hydroxy-1,8-Naphtyridinen Va, b umlagert.

Die Darstellung der β-Ketoester II kann wie in Organic Synthesis Coll., Vol. 1, S. 248, oder in DE-A- 3 227 388 beschrieben, durchgeführt werden.

2-Amino-pyridine der Formel III sind in Beilstein, Handbuch der Organ. Chemie, Band E I 22, S. 633ff., Band E II 22, S. 342ff. sowie Band E III/IV 22, S. 4133ff. aufgeführt.

Die Umsetzung der β-Ketoester II mit den 2-Amino-pyridinen III kann vorteilhaft in einem die Kondensation erleichternden Lösungsmittel durchgeführt werden. Insbesondere kommen Polyphosphorsäure oder Gemische aus Polyphosphorsäure und Phosphoroxytrichlorid bzw. Thionylchlorid, gegebenenfalls verdünnt mit einem inerten Solvens wie Toluol oder Xylol, in Frage. Die Reaktionstemperatur liegt i.a. zwischen 80 und 200 °C, vorzugsweise bei 100 bis 150 °C. Die Kondensationsprodukte werden isoliert, indem man die Reaktionsmischung, nach Abdampfen von gegebenenfalls vorhandenem inerten Solvens wie Toluol oder Xylol, durch Zugabe einer wäßrigen Lösung eines Alkali- oder Erdalkalihydroxids wie Natrium-, Kalium-oder Calciumhydroxid neutralisiert, mit Wasser den entstandenen Niederschlag phosphatfrei wäscht und den verbliebenen Rückstand sorgfältig trocknet.

Eine Reinigung durch Kristallisation aus Solventien wie Pentan, Petrolether oder Ligroin kann gegebenenfalls angeschlossen werden.

Die Umlagerung der Kondensationsprodukte IV zu den 4-Hydroxynaphthyridinen Va, b wird in einem hochsiedenden, inerten Lösungsmittel wie Paraffin-Öl, Diphenyl, Diphenylether, Diphenyl/Diphenylether 1:1, Weißöl o.ä. bei Temperaturen zwischen 200 und 350 °C, vorzugsweise bei 250 bis 300 °C, vorgenommen. Durch Einrühren der erkalteten Reaktionsmischung in Lösungsmittel wie Pentan, Petrolether, Cyclohexan oder Ligroin wird das Produkt gefällt und durch Filtration abgetrennt. Eine weitere Reinigung ist meist nicht erforderlich.

Die 4-Hydroxy-naphthyridine Va, b lassen sich durch Umsetzung mit Phosphorhalogeniden oder Schwefelhalogeniden, vorzugsweise Phosphoroxytrichlorid oder Thionylchlorid, halogenieren. Hierbei kann das Halogenierungsmittel im Überschuß als Solvens oder in (nahezu) äquimolaren Mengen in Gegenwart eines Solvens wie Benzol, Toluol, Xylol; Chlorbenzol, o-Dichlorbenzol, Nitrobenzol; 1,1-Dichlorethan, 1,2-Dichlorethan, 1,1,1-Trichlorethan oder 1,1,2-Trichlorethylen mit der Hydroxy-Verbindung zur Reaktion gebracht werden. Eine Base, z.B. Triethylamin oder N,N-Dimethylanilin, kann in stöchiometrischer Menge oder im Überschuß zugesetzt werden. Die Reaktionstemperatur liegt zwischen 50 und 200 °C, vorzugsweise 80 bis 120 °C. Naoh Verdampfen von überschüssigem Halogenierungsmittel bzw. Solvens wird der Rückstand mit Eiswasser, ggf. unter Zusatz eines mit Wasser nicht mischbaren Lösungsmittels, behandelt und ggf. die Base durch Extraktion mit Säure entfernt. Das so gewonnene Chlorierungsprodukt bedarf meist keiner weiteren Reinigung.

Weiterhin lassen sich die 4-Hydroxy-naphthyridine Va, b durch Umsetzung mit Schwefelwasserstoff, Phosphorpentasulfid oder 2,4-Bis-(4-methoxy-phenyl)-1,3-dithia-2,4-diphosphetan-2,4-disulfid (p-Methoxyphenyl-thionophosphin-sulfid) IX

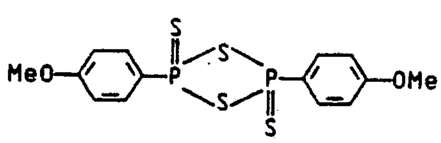

IX

(siehe z.B. W. Walter et al., Synthesis 1979, 941) in die entsprechenden 4-Mercapto-Verbindungen VI (X = SH) überführen.

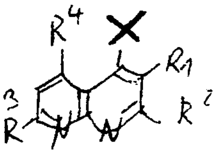

VI

Als Lösungsmittel können Pyridin, Picoline, Lutidine, Collidine; 1,2-Dimethoxy-ethan, Tetrahydrofuran, 1,4-Dioxan; Hexamethylphosphorsäuretriamid; Tetramethylharnstoff, N,N-Dimethylethylenharnstoff oder N,N-Dimethyl-propylenharnstoff verwendet werden.

Daran anschließend läßt sich die S-Alkylierung der so gewonnenen 4-Mercapto-Verbindungen zu den entsprechenden 4-Alkylthio-Verbindungen VI (X = S-Alkyl) vornehmen. Als Alkylierungsmittel seien Alkylhalogenide wie Methyl- oder Ethylchlorid, Methyl- oder Ethylbromid, Alkyl-tosylate oder -mesylate wie p-Tolylsulfonsäure- oder Methansulfonsäuremethylester; Dialkylsulfate wie Dimethyl- oder Diethylsulfat genannt; ggf. kann unter Zusatz einer Base wie Natrium- oder Calciumcarbonat; Natrium- oder Kaliumhydroxid, ggf. unter Verwendung eines Solvens wie Methanol, Ethanol oder Butanol; Aceton oder 2-Butanon; N,N-Dimethylformamid oder N-Methylpyrrolidon bei Temperaturen zwischen +20 und +150°C gearbeitet werden.

Danach kann die Oxidation der so erhaltenen 4-Alkylthio-Verbindungen in die entsprechenden 4-Alkylsulfinyl- bzw. 4-Alkyl-sulfonyl-Verbindungen VI (X = S0-Alkyl bzw. $S0_2$-Alkyl) vorgenommen werden, indem man sie mit einem bzw. zwei Äquivalenten eines Oxidationsmittels reagieren läßt. Als Oxidationsmittel kommen in Frage: Wasserstoff-peroxid; Peressigsäure, Trifluorperessigsäure, Perbenzoesäure, m-Chlorperbenzoesäure; tert.-Butyl-hydroperoxid, N-Methyl-morpholin-N-oxid; Kaliumpersulfat, Kaliumperoxodisulfat oder Natriumperborat; als Lösungsmittel: Wasser, Essigsäure, Trifluoressigsäure; Methylenchlorid, 1,2-Dichlor-ethan, 1,1,1-Trichlorethan odr Chlorbenzol. Die Reaktionstemperatur liegt zwischen 0 und +150°C, vorzugsweise zwischen 40 und 80°C, mit oder ohne Katalysator.

Durch Reaktion mit Hydrazinen der Formel $NH_2$-$NR^5R^6$ wie Hydrazin, Methylhydrazin, 1,1-Dimethylhydrazine; Formyl- oder Acetyl-hydrazin können die genannten 4-Halogen-Verbindungen VI (X = Halogen), 4-Mercapto-Verbindungen VI (X = SH), 4-Alkylthio-Verbindungen VI (X = Alkyl-S), 4-Alkyl-sulfinyl-Verbindungen VI (X = Alkyl-S0) bzw. 4-Alkyl-sulfonyl-Verbindungen VI (X = Alkyl-$S0_2$) in die entsprechenden 4-Hydrazino-Verbindungen VII umgewandelt werden. Als Lösungsmittel kommen Ether wie Di-n-butyl-ether, Diethylenglykol-dimethyl-oder -diethylether; Alkohole wie Butanol, 2-Ethylhexanol der Ethylenglykol; N,N-Dimethylformamid, N-Methylpyrrolidon oder Dimethylsulfoxid in Frage. Die Reaktionstemperatur liegt zwischen +80°C und +200°C.

Die genannten 4-Hydrazino-Verbindungen VII u lassen sich mit einem geeigneten Reduktionsmittel in die entsprechenden 4-Amino-Verbindungen I (Z = $NH_2$) umwandeln. Als Reduktionsmittel kommen Wasserstoff in Anwesenheit eines Katalysators wie Nickel, Palladium oder Platin, ggf. dispergiert auf einem inerten Träger wie Aktivkohle, Kieselgur oder Aluminiumoxid, oder Natriumdithionit (Natriumhydrosulfit) in Frage, als Lösungsmittel Alkohole wie Methanol, Ethanol, Butanol; Carbonsäuren wie Ameisen- oder Essigsäure; Ether wie Tetrahydrofuran oder 1,4-Dioxan, ggf. im Gemisch mit Wasser, oder im Falle des Natriumdithionits, wäßrige Alkali-Lösungen wie Natron- oder Kalilauge, ggf. unter Zusatz eines Lösungsvermittlers wie Ethanol, 1,4-Dioxan oder Ethylenglykol.

Herstellungsbeispiel

4-Amino-2,7-dimethyl-3-n-octyl-1,8-naphthyridin

a) 3,5-Dimethyl-2-n-octyl-4H-pyrido[1,2-a]-pyrimidin-4-on (Beispiel Nr. 13 A)

10,8 g (100 mmol) 2-Amino-6-methyl-pyridin werden unter Rühren zu 100 g Polyphosphorsäure zugegeben. Man heizt binnen 2 h auf 120°C und tropft dabei 22,8 g (100 mmol) 2-n-Octyl-acetessigsäure-methylester zu. Es wird noch 30 min nachgerührt; danach läßt man abkühlen. Zur Reaktionsmischung gibt man anschließend 100 ml Wasser und läßt über Nacht rühren. Die entstandene einheitliche Lösung wird mit einer Lösung von 75 g Natriumhydroxid in 250 ml Wasser unter Kühlung versetzt und so lange bei Raumtemperatur (20°C) gerührt, bis die Bildung eines gelben Niederschlags beendet ist. Letzterer wird

abgesaugt, intensiv mit 1,5 l Wasser nachgewaschen, trocken gesaugt und sorgfältig getrocknet. Ausbeute 23,9 g (83 %) eines nach Dünnschichtchromatogramm einheitlichen Pulvers mit Schmp. 42-45° C. Umlösen aus Petrolether ergibt nahezu farblose Kristalle mit Schmp. 56-58° C.

b) 4-Hydroxy-2,7-dimethyl-3-n-octyl-1,8-naphthyridin (Beispiel Nr. 13 B)

Man erhitzt 250 ml dickflüssiges Paraffinöl auf 300° C und setzt in kleinen Portionen 14,3 g (50,0 mmol) Produkt aus a) zu. Nach einer Reaktionszeit von 30 min. läßt man abkühlen, gibt 250 ml Petrolether zu, rührt 1,5 h nach und saugt den entstandenen Niederschlag ab. Nach Nachwaschen und Trocknen fallen 5,72 g (40 %) graues Pulver mit Schmp. 215-216° C an. Die Substanz liegt laut IR-Spektren nahezu vollständig in der 1H-4-Keto-Form vor.

c) 4-Chlor-2,7-dimethyl-3-n-octyl-1,8-naphthyridin (Beispiel Nr. 13 C)

58,0 g (203 mmol) Produkt aus b) werden in 400 ml Phosphoroxytrichlorid 18 h lang unter Rückfluß erhitzt. Nach Abkühlen wird überschüssiges Chlorierungsreagens abdestilliert. Den Rückstand löst man in Dichlormethan, wäscht mit verd. Natriumhydrogencarbonat-Lösung und Wasser, trocknet und engt die Lösung ein. Man reibt den Rückstand mit Pentan an. Ausbeute 42 g (68 %), Schmp. 68-69° C, dünnschichtchromatographisch einheitlich.

d) 4-Hydrazino-2,7-dimethyl-3-n-octyl-1,8-naphthyridin (Beispiel Nr. 13 D)

43,0 g (141 mmol) Produkt aus c) werden 12 h lang in 400 ml Hydrazinhydrat unter Rückfluß erhitzt. Nach Abkühlen saugt man das ausgefallene Material ab, löst in Dichlormethan, wäscht dreimal mit Wasser, trocknet und engt ein. Der Rückstand wird mit Pentan verrieben, abgesaugt und getrocknet. Ausbeute 33,4 g (79 %), Schmp. 103-104° C.

e) 4-Amino-2,7-dimethyl-3-n-octyl-1,8-naphthyridin (Beispiel Nr. 13 E)

33,4 g (111 mmol) Produkt aus d) werden in 600 ml 10 %iger wäßriger Natronlauge suspendiert. Man heizt auf 65° C, gibt ca. 300 ml Ethanol zu, bis eine klare Lösung entstanden ist, läßt auf 45° C abkühlen und fügt 19,7 g (113 mmol) Natriumdithionit hinzu. Danach erhitzt man 12 h unter Rückfluß. Bei Abkühlen entsteht ein Niederschlag, der abgesaugt, mit Wasser gewaschen und trocken gesaugt wird. Man behandelt nun mit Pentan, saugt nochmals ab und trocknet. Ausbeute 23,8 g (75 %), Schmp. 176-178° C.

Nach den angegebenen Verfahren wurden die in den nachstehenden Tabellen näher charakterisierten Wirkstoffe hergestellt. Die nicht näher charakterisierten Verbindungen können unter entsprechender Abwandung der Rohstoffe und Anpassung der Herstellvorschriften leicht erhalten werden; sie lassen aufgrund ihrer strukturellen Ähnlichkeit eine vergleichbare Wirkung erwarten.

Die in den Tabellen verwendeten Abkürzungen bedeuten:

A: 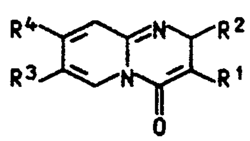 (Vorprodukt)

B: 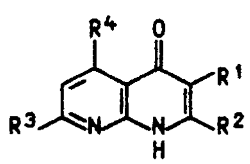 (Vorprodukt)

C: 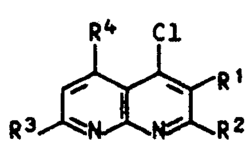 (Vorprodukt)

D: 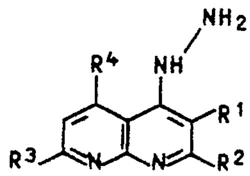 (Endprodukt bzw. Vorprodukt für E)

E: 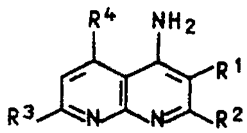 (Endprodukt)

Tabelle

| Nr. | $R^1$ | $R^2$ | $R^3$ | $R^4$ | A Schmp. (°C) | B Schmp. (°C) | C Schmp. (°C) | D Schmp. (°C) | E Schmp. (°C) |
|---|---|---|---|---|---|---|---|---|---|
| 1 | $n-C_4H_9$ | $CH_3$ | $CH_3$ | H | | | | | |
| 2 | $n-C_4H_9$ | $CH_3$ | $CH_3$ | $CH_3$ | | | | | |
| 3 | $i-C_4H_9$ | $CH_3$ | $CH_3$ | H | | | | | |
| 4 | $i-C_4H_9$ | $CH_3$ | $CH_3$ | $CH_3$ | | | | | |
| 5 | $n-C_5H_{11}$ | $CH_3$ | $CH_3$ | H | 54-55 | 217-218 | 84-85 | 157-158 | 207-208 |
| 6 | $n-C_5H_{11}$ | $CH_3$ | $CH_3$ | $CH_3$ | | | | | |
| 7 | $i-C_5H_{11}$ | $CH_3$ | $CH_3$ | H | | | | | |
| 8 | $i-C_5H_{11}$ | $CH_3$ | $CH_3$ | $CH_3$ | | | | | |
| 9 | $n-C_6H_{13}$ | $CH_3$ | $CH_3$ | H | 50-51 | 227-228 | 68-69 | 152-153 | 193-194 |
| 10 | $n-C_6H_{13}$ | $CH_3$ | $CH_3$ | $CH_3$ | | | | | |
| 11 | $n-C_7H_{15}$ | $CH_3$ | $CH_3$ | H | | | | | |
| 12 | $n-C_7H_{15}$ | $CH_3$ | $CH_3$ | $CH_3$ | | | | | |
| 13 | $n-C_8H_{17}$ | $CH_3$ | $CH_3$ | H | 56-58 | 215-216 | 68-69 | 103-104 | 176-178 |
| 14 | $n-C_8H_{17}$ | $CH_3$ | $CH_3$ | $CH_3$ | | | | | |
| 15 | $n-C_9H_{19}$ | $CH_3$ | $CH_3$ | H | | | | | |
| 16 | $n-C_9H_{19}$ | $CH_3$ | $CH_3$ | $CH_3$ | | | | | |
| 17 | $n-C_{10}H_{21}$ | $CH_3$ | $CH_3$ | H | | | | | |
| 18 | $n-C_{10}H_{21}$ | $CH_3$ | $CH_3$ | $CH_3$ | | | | | |
| 19 | $n-C_{11}H_{23}$ | $CH_3$ | $CH_3$ | $CH_3$ | | | | | |
| 20 | $n-C_{12}H_{25}$ | $CH_3$ | $CH_3$ | $CH_3$ | | | | | |
| 21 | $(n-C_3H_7)(CH_3)CHCH_2$ | $CH_3$ | $CH_3$ | H | | | | | |
| 22 | $(n-C_3H_7)(CH_3)CHCH_2$ | $CH_3$ | $CH_3$ | $CH_3$ | | | | | |

Tabelle: Fortsetzung

| Nr. | $R^1$ | $R^2$ | $R^3$ | $R^4$ | A Schmp. (°C) | B Schmp. (°C) | C Schmp. (°C) | D Schmp. (°C) | E Schmp. (°C) |
|---|---|---|---|---|---|---|---|---|---|
| 23 | $(C_2H_5)(CH_3)CHCH_2$ | $CH_3$ | $CH_3$ | H | | | | | |
| 24 | $(C_2H_5)(CH_3)CHCH_2$ | $CH_3$ | $CH_3$ | $CH_3$ | | | | | |
| 25 | $(C_2H_5)_2CHCH_2$ | $CH_3$ | $CH_3$ | H | | | | | |
| 26 | $(C_2H_5)_2CHCH_2$ | $CH_3$ | $CH_3$ | $CH_3$ | | | | | |
| 27 | $(n\text{-}C_4H_9)(C_2H_5)CHCH_2$ | $CH_3$ | $CH_3$ | H | | | | | |
| 28 | $(n\text{-}C_4H_9)(C_2H_5)CHCH_2$ | $CH_3$ | $CH_3$ | $CH_3$ | | | | | |
| 29 | $t\text{-}C_4H_9CH_2(CH_3)CHCH_2$ | $CH_3$ | $CH_3$ | H | | | | | |
| 30 | $t\text{-}C_4H_9CH_2(CH_3)CHCH_2$ | $CH_3$ | $CH_3$ | $CH_3$ | | | | | |
| 31 | $t\text{-}C_4H_9CH_2(CH_3)CH(CH_2)_2$ | $CH_3$ | $CH_3$ | H | Öl | 204-207 | 79-82 | 100-104 | Harz |
| 32 | $t\text{-}C_4H_9CH_2(CH_3)CH(CH_2)_2$ | $CH_3$ | $CH_3$ | $CH_3$ | | | | | |
| 33 | $(n\text{-}C_4H_9)(C_2H_5)CH—CH_2$<br>$H_2C—CH(CH_3)—O$ | $CH_3$ | $CH_3$ | H | | | | | |
| 34 | $(n\text{-}C_4H_9)(C_2H_5)CH—CH_2$<br>$CH_2—HC(CH_3)—O$ | $CH_3$ | $CH_3$ | $CH_3$ | | | | | |
| 35 | $t\text{-}C_4H_9CH_2CH(CH_3)(CH_2)_2$<br>$CH_2—CH(CH_3)—O$ | $CH_3$ | $CH_3$ | H | | | | | |
| 36 | $t\text{-}C_4H_9CH_2CH(CH_3)(CH_2)_2$<br>$CH_2—CH(CH_3)—O$ | $CH_3$ | $CH_3$ | $CH_3$ | | | | | |
| 37 | $(C_2H_5)_2CHCH_2—O$<br>$H_2C—CH(CH_3)$ | $CH_3$ | $CH_3$ | H | | | | | |
| 38 | $(C_2H_5)_2CHCH_2—O$<br>$H_2C—CH(CH_3)$ | $CH_3$ | $CH_3$ | $CH_3$ | | | | | |

Die neuen Wirkstoffe zeigen eine starke fungitoxische Wirksamkeit gegen phytopathogene Pilze, insbesondere aus der Klasse der Phycomyceten. Sie sind daher zur Bekämpfung von beispielsweise Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais,

Bremia lactucae an Salat, Mucor mucedo an Früchten, Rhizopus nigricans an Rüben sowie ferner von Erysiphe graminis an Getreide, Uncinula necator an Reben, Podosphaera leucotricha an Äpfeln und Pyrenophora teres an Gerste geeignet.

Die Wirkstoffe weisen eine hohe Pflanzenverträglichkeit auf. Ein Teil der Wirkstoffe zeigt kurative Eigenschaften, d.h. die Anwendung der Mittel kann noch nach der Pilzinfektion der Pflanzen durch die Krankheitserreger vorgenommen werden, um einen Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die neuen Wirkstoffe werden beispielsweise in Form von direkt versprühbaren Lösungen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten oder Öldispersionen aus Mineralölfraktionen von mittlerem bis hohem Siedepunkt, wie Kerosin oder Dieselöl, ferner Kohlenteerölen usw., sowie Öle pflanzlichen oder tierischen Ursprungs, aliphatische, cyclische oder aromatische Kohlenwasserstoffe, z.B. Benzyl, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten, oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiemittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfate, Fettalkoholsulfate, fettsaure Alkali- und Erdalkalisalze, Salze sulfatierter Hexadecanole, Heptadecanole, Octadecanole, Salze von sufatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolalkohol, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Attaclay, Kalkstein, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Zubereitungsbeispiele für Pflanzenschutzmittel:

I. Man vermischte 90 Gew.-Teile der Verbindung des Beispiels 9 mit 100 Gew.-Teilen N-Methylpyrrolidon und erhielt eine Lösung, die zur Anwendung in Form kleinster Tropfen geeignet war.

II. 20 Gew.-Teile der Verbindung des Beispiels 13 wurden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des

Anlagerungsproduktes und 40 Mol Ethylenoxid an 1 Mol Ricinusöl bestand. Durch Ausgießen und feines Verteilen der Lösung in Wasser erhielt man eine wäßrige Dispersion.

III. 20 Gew.-Teile der nach Beispiel 13 erhältlichen Verbindung wurden in einer Mischung gelöst, die aus 30 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl bestand. Durch Eingießen und Verrühren der Lösung in Wasser erhielt man eine wäßrige Dispersion.

IV. 20 Gew.-Teile der nach Beispiel 9 erhältlichen Verbindung wurden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl bestand. Durch Eingießen und Verrühren der Lösung in Wasser erhielt man eine wäßrige Dispersion.

V. 20 Gew.-Teile der nach Beispiel 13 erhältlichen Verbindung wurden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in Wasser erhielt man eine Spritzbrühe.

VI. 5 Gew.-Teile der nach Beispiel 9 erhältlichen Verbindung wurden mit 95 Gew. -Teilen feinteiligem $\gamma$ olin innig vermischt. Man erhielt auf diese Weise ein Stäubemittel, das 5 Gew.% des Wirkstoffs enthielt.

VII.30 Gew.-Teile der nach Beispiel 13 erhältlichen Verbindung wurden mit einer Mischung aus 92 Gew.-Teilen pulverförmigem Kieselsäuregel und 8 Gew. -Teilen Paraffinöl, das auf die Oberfläche dieses Kieselsäuregels gesprüht wurde, innig vermischt. Man erhielt auf diese Weise eine Aufbereitung des Wirkstoffs mit guter Haftfähigkeit.

VIII.40 Gew.-Teile der nach Beispiel 13 erhältlichen Verbindung wurden mit 30 Gew. -Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhielt eine stabile wäßrige Dispersion.

IX. 20 Gew. -Teile der nach Beispiel 9 erhältlichen Verbindung wurden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhielt eine stabile ölige Dispersion.

Für die folgenden Anwendungsbeispiele wurde als bekannte Vergleichs-Verbindung das N-Trichlormethylthio-tetrahydrophthalimid A (Chem. Week, June 21, 1972, Seite 46) verwendet.

Anwendungsbeispiel 1

Wirksamkeit gegen Phytophthora infestans an Tomaten

Blätter von Topfpflanzen der Sorte "GroBe Fleischtomate" wurden mit wäßriger Spritzbrühe, die 80 Gew.% Wirkstoff und 20 Gew.% Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18 °C aufgestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis des Versuches zeigt, daß bei der Anwendung als 0,025 %ige (Gew.%) Spritzbrühe beispielsweise die Verbindung 13E eine bessere fungizide Wirkung zeigt (97 %) als der bekannte Wirkstoff A (90 %).

Anwendungsbeispiel 2

Wirksamkeit gegen Plasmopara viticola

Blätter von Topfreben der Sorte "Müller-Thurgau" wurden mit wäßriger Spritzbrühe, die 80 Gew.% Wirkstoff und 20 Gew.% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigt, daß bei der Anwendung als 0,05 %ige Spritzbrühe beispielsweise

die Verbindungen 9E, 13D und 13E eine gute fungizide Wirkung zeigen (100 %).

**Patentansprüche**

1. 1,8-Naphthyridin-Derivate der Formel I,

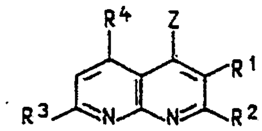

I

worin

$R^1$ einen $C_4$- bis $C_{12}$-Alkyl- oder $C_4$- bis $C_{12}$-Alkoxy-$C_1$-$C_4$-alkylrest bedeutet,
$R^2$ und $R^3$ unabhängig voneinander $C_1$- bis $C_3$-Alkyl,
$R^4$ Wasserstoff oder $C_1$-$C_4$-Alkyl,
Z $NH_2$ oder NH-$NR^6R^5$,
$R^5$ und $R^6$ unabhängig voneinander Wasserstoff, $C_1$-$C_4$-Alkyl, Formyl oder $C_1$-$C_4$-Alkyl-CO

bedeuten.

2. Verfahren zur Herstellung von substituierten 1,8-Naphthyridinen der Formel I gemäß Anspruch 1, dadurch gekennzeichnet, daß man einen $\beta$-Ketoester der Formel II

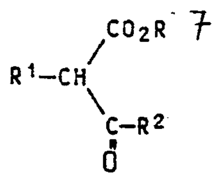

II,

in der $R^7$ für einen $C_1$-$C_4$-Alkylrest steht, mit einem 2-Amino-pyridin der Formel III

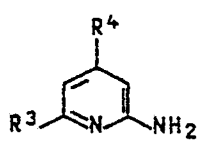

III,

wobei $R^1$ bis $R^4$ die in Anspruch 1 genannte Bedeutung besitzen, zu einem Kondensationsprodukt der Formel IV

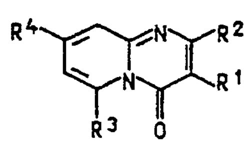

IV,

umsetzt, dieses zum Isomeren der Formel Va, b

Va 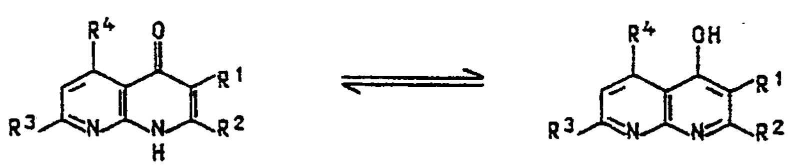 Vb

umlagert, dieses mit Schwefelwasserstoff, Phosphorpentasulfid oder 2,4-Bis-(4-methoxy-phenyl)-1,3-dithia-2,4-diphosphetan-2,4-disulfid (p-Methoxy-phenyl-thionophosphinsulfid) der Formel

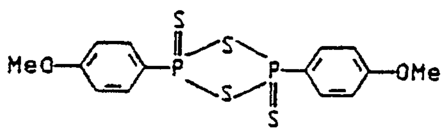

in das Derivat VI

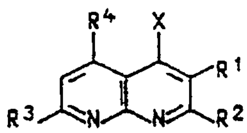

VI.

üerführt, wobei X die Bedeutung Halogen, Mercapto, $C_1$-$C_4$-Alkylthio, $C_1$-$C_4$-Alkylsulfinyl oder $C_1$-$C_4$-Alkylsulfonyl hat, und dieses mit $NH_2$-$NR^6R^5$ zu Verbindungen I gemaß Anspruch 1 umsetzt mit Z = -NH-$NR^5R^6$ und diese gegebenenfalls zu der Aminoverbindung (Z = $NH_2$) reduziert.

**3.** Fungizid gegen pflanzenphathogene Pilze, gekennzeichnet durch einen, Gehalt an einem Trägerstoff und einem 1,8-Naphthyridin-Derivat entsprechend Anspruch 1 der Formel I

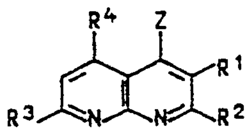

I

worin

| | |
|---|---|
| $R^1$ | einen $C_4$- bis $C_{12}$-Alkyl- oder $C_4$- bis $C_{12}$-Alkoxy-$C_1$-$C_4$-alkylrest bedeutet, |
| $R^2$ | und $R^3$ unabhängig voneinander $C_1$- bis $C_3$-Alkyl, |
| $R^4$ | Wasserstoff oder $C_1$-$C_4$-Alkyl, |
| Z | $NH_2$ oder NH-$NR^6R^5$, |
| $R^5$ und $R^6$ | unabhängig voneinander Wasserstoff, $C_1$-$C_4$-Alkyl, Formyl oder $C_1$-$C_4$-Alkyl-CO |

bedeuten.

**4.** Verfahren zur Bekämpfung von pflanzenpathogenen Pilzen, dadurch gekennzeichnet, daß man die Pilze oder die vor Pilzbefall zu schützenden Materialien, Pflanzen, Boden oder Saatgüter mit einer fungizid wirksamen Menge eines 1,8-Naphthyridin-Derivats entsprechend Anspruch 1 der Formel I

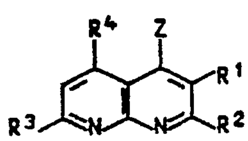

I

worin

| | |
|---|---|
| $R^1$ | einen $C_4$- bis $C_{12}$-Alkyl- oder $C_4$- bis $C_{12}$-Alkoxy-$C_1$-$C_4$-alkylrest bedeutet, |
| $R^2$ | und $R^3$ unabhängig voneinander $C_1$- bis $C_3$-Alkyl, |
| $R^4$ | Wasserstoff oder $C_1$-$C_4$-Alkyl. |
| Z | $NH_2$ oder NH-$NR^6R^5$, |
| $R^5$ | und $R^6$ unabhängig voneinander Wasserstoff, $C_1$-$C_4$-Alkyl, Formyl oder $C_1$-$C_4$-Alkyl-CO |

bedeuten, behandelt.

**5.** 4-Amino-2,7-dimethyl-3-n-octyl-1-1,8-naphthyridin.

6. 4-Amino-2,7-dimethyl-3-n-hexyl-1-1,8-naphthyridin.

**Claims**

1. A 1,8-naphthyridine derivative of the formula I

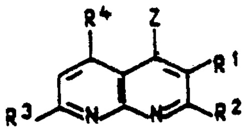

I

where $R^1$ is $C_4$-$C_{12}$-alkyl or $C_4$-$C_{12}$-alkoxy-$C_1$-$C_4$-alkyl, $R^2$ and $R^3$ independent of one another are each $C_1$-$C_3$-alkyl, $R^4$ is hydrogen or $C_1$-$C_4$-alkyl, Z is $NH_2$ or NH-$NR^6R^5$ and $R^5$ and $R^6$ independently of one another are each hydrogen, $C_1$-$C_4$-alkyl, formyl or $C_1$-$C_4$-alkyl-CO.

2. A process for the preparation of a substituted 1,8-naphthyridine of the formula I as claimed in claim 1, wherein a $\beta$-keto ester of the formula II

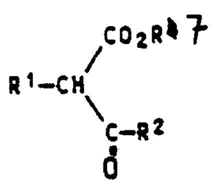

II

where $R^7$ is $C_1$-$C_4$-alkyl, is reacted with a 2-aminopyridine of the formula III

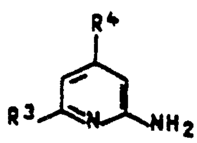

III

where $R^1$ to $R^4$ have the meanings stated in claim 1, to give a condensate of the formula IV

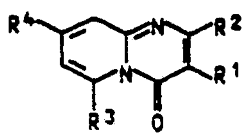

IV

the latter is subjected to a rearrangement reaction to give isomers of the formulae Va and Vb

Va 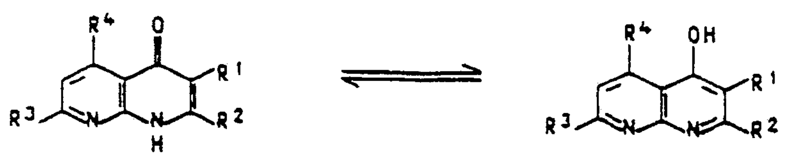 Vb

these are converted with hydrogen sulfide, phosphorus pentasulfide or 2,4-bis-(4-methoxyphenyl)-1,3-dithia-2,4-diphosphetane 2,4-disulfide (p-methoxyphenylthionophosphine sulfide) of the formula

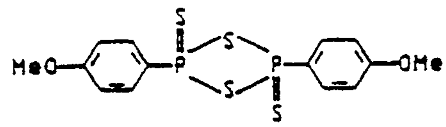

into a derivative VI

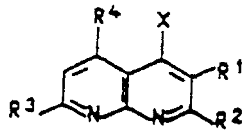

VI

where X is halogen, mercapto, $C_1$-$C_4$-alkylthio, $C_1$-$C_4$-alkylsulfinyl or $C_1$-$C_4$-alkylsulfonyl, and the latter is reacted with $NH_2$-$NR^6R^5$ to give a compound I as claimed in claim 1, in which Z is -NH-$NR^5R^6$, and, if required, the latter is reduced to the amino compound (Z = $NH_2$).

**3.** A fungicide against phytopathogenic fungi, which contains a carrier and a 1,8-naphthyridine derivative as claimed in claim 1, of the formula I

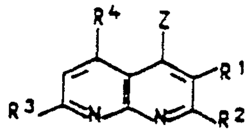

I

where $R^1$ is $C_4$-$C_{12}$-alkyl or $C_4$-$C_{12}$-alkoxy-$C_1$-$C_4$-alkyl, $R^2$ and $R^3$ independently of one another are each $C_1$-$C_3$-alkyl, $R^4$ is hydrogen or $C_1$-$C_4$-alkyl, Z is $NH_2$ or NH-$NR^6R^5$ and $R^5$ and $R^6$ independently of one another are each hydrogen, $C_1$-$C_4$-alkyl, formyl or $C_1$-$C_4$-alkyl-CO.

**4.** A method for controlling phytopathogenic fungi, wherein the fungi or the materials, plants, soil or seeds to be protected from fungal attack are treated with a fungicidal amount of a 1,8-naphthyridine derivative as claimed in claim 1, of the formula I

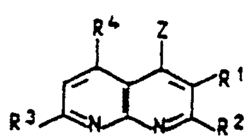

I

where $R^1$ is $C_4$-$C_{12}$-alkyl or $C_4$-$C_{12}$-alkoxy-$C_1$-$C_4$-alkyl, $R^2$ and $R^3$ independently of one another are each $C_1$-$C_3$-alkyl, $R^4$ is hydrogen or $C_1$-$C_4$-alkyl, Z is $NH_2$ or NH-$NR^6R^5$ and $R^5$ and $R^6$ independently of one another are each hydrogen, $C_1$-$C_4$-alkyl, formyl or $C_1$-$C_4$-alkyl-CO.

**5.** 4-Amino-2,7-dimethyl-3-n-octyl-1,8-naphthyridine.

**6.** 4-Amino-2,7-dimethyl-3-n-hexyl-1,8-naphthyridine.

## Revendications

**1.** 1,8-naphtyridines de formule I

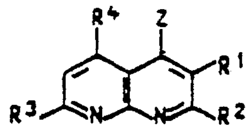

I

dans laquelle

$R^1$ représente un groupe alkyle en C 4-6 12 ou (alcoxy en C 4-6 12)-alkyle en C 1-C 4,

$R^2$ et $R^3$ représentent chacun, indépendamment l'un de l'autre, un groupe alkyle en C 1-C 3,

$R^4$ représente l'hydrogène ou un groupe alkyle en C 1-C 4,

Z représente $NH_2$ ou NH-$NR^6R^5$,

$R^5$ et $R^6$ représentent chacun, indépendamment l'un de l'autre, l'hydrogène, un groupe alkyle en C 1-C 4, formyle, ou (alkyle en C 1-C 4)-CO,

**2.** Procédé de préparation des 1,8-naphtyridines substituées de formule I de la revendication 1, caractérisé en ce que l'on fait réagir un bêta-cétoester de formule II

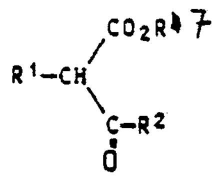

II,

dans laquelle $R^7$ représente un groupe alkyle en C 1-C 4, avec une 2-aminopyridine de formule III

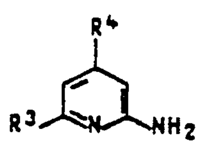

III,

dans laquelle $R^1$ à $R^4$ ont les significations indiquées dans la revendication 1, ce qui donne un produit de condensation de formule IV

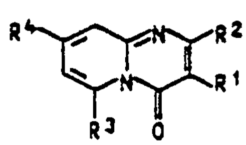

IV,

qu'on transpose en l'isomère de formule Va, b

Va

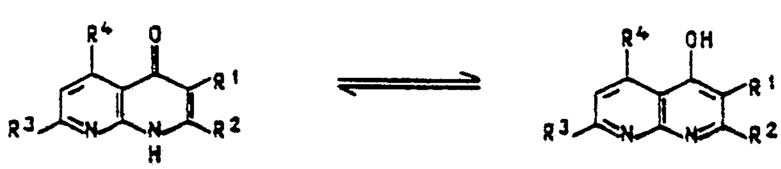

Vb

qu'on convertit, à l'aide du sulfure d'hydrogène, du pentasulfure de phosphore ou du 2,4-disulfure de 2,4-bis-(4-méthoxyphényl)-1,3-dithia-2,4-diphosphétane (sulfure de p-méthoxyphényl-thionophosphine) de formule

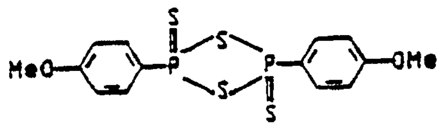

en le dérivé de formule VI

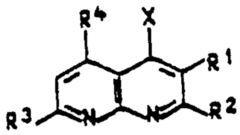

VI.

dans laquelle X représente un halogène, un groupe mercapto, alkylthio en C 1-C 4, alkylsulfinyle en C 1-C 4 ou alkylsulfonyle en C 1-C 4, après quoi on convertit ce dérivé, par réaction avec $NH_2$-$NR^6R^5$, en composés de formule I de la revendication 1 dans laquelle Z = -NH-$NR^5R^6$, qu'on réduit le cas échéant en le dérivé aminé (Z = $NH_2$).

3. Produit fongicide à l'égard des mycètes phytopathogènes, caractérisé en ce qu'il contient un véhicule et une 1,8-naphtyridine de formule I selon la revendication 1

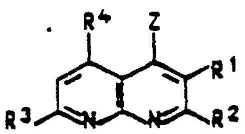

I

dans laquelle

$R^1$ représente un groupe alkyle en C 4-C 12 ou (alcoxy en C 4-C 12)-alkyle en C 1-C 4,

$R^2$ et $R^3$ représentent chacun, indépendamment l'un de l'autre, un groupe alkyle en C 1-C 3,

$R^4$ représente l'hydrogène ou un groupe alkyle en C 1-C 4,

Z représente $NH_2$ ou NH-$NR^6R^5$,

$R^5$ et $R^6$ représentent chacun, indépendamment l'un de l'autre, l'hydrogène, un groupe alkyle en C 1-C 4, formyle ou (alkyle en C 1-6 4)-CO.

4. Procédé pour combattre les mycètes phytopathogènes, caractérisé en ce que l'on traite les mycètes ou les matières, plantes, sols ou semences à protéger contre l'infection par les mycètes, par une quantité fongicide efficace d'une 1,8-naphtyridine selon la revendication 1, répondant à la formule I

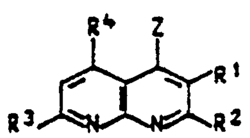

I

dans laquelle

$R^1$ représente un groupe alkyle en C 4-C 12 ou (alcoxy en C 4-C 12)-alkyle en C 1-C 4,

$R^2$ et $R^3$ représentent chacun, indépendamment l'un de l'autre, un groupe alkyle en C 1-C 3,

$R^4$ représente l'hydrogène ou un groupe alkyle en C 1-C 4,

Z représente $NH_2$ ou $NH-NR^6R^5$,

$R^5$ et $R^6$ représentent chacun, indépendamment l'un de l'autre, l'hydrogène, un groupe alkyle en C 1-C 4, formyle ou (alkyle en C 1-C 4)-CO.

**5.** La 4-amino-2,7-diméthyl-3-n-octyl-1,8-naphtyridine.

**6.** La 4-amino-2,7-diméthyl-3-n-hexyl-1,8-naphtyridine.